# EUROPEAN PATENT APPLICATION

(11) **EP 3 646 848 A1**
(43) Date of publication of application: **06.05.2020**
(21) Application number: 18203091.6
(22) Date of filing: 29.10.2018
(51) Int. Cl.: A61K 8/34, A61K 8/55, A61Q 17/00, A61Q 19/00, A61K 8/81, A61K 8/92

(54) **COMPOSITIONS FOR DRYING AND CARE OF SKIN**

(71) Applicant: Miverde AG, 6300 Zug (CH)
(72) Inventor: Eberl-Wallimann, Joachim, 6005 Luzern (CH)
(74) Representative: Strehl Schübel-Hopf & Partner

(57) **Abstract**

The present invention relates to compositions, for example in the form of semisolid waxes, creams, lotions or gels, which are capable of drying and caring of skin and, if required, show disinfecting properties. The present invention also relates to the use of these compositions for drying or drying and disinfecting the skin, and to a method of washing and drying hands. The compositions of the present invention comprise a combination of typical cosmetic components and contain a high content of a gelling agent and a comparatively low or no content of water.

## Description

The present invention relates to compositions, for example in the form of semisolid waxes, creams, lotions or gels, which are capable of drying and care of skin and, if required, show disinfecting properties. The present invention also relates to the use of these compositions for drying or drying and disinfecting the skin, and to a method of washing and drying hands. The compositions of the present invention comprise a combination of typical cosmetic components and contain a high content of a gelling agent and a comparatively low or no content of water. The compositions of the present invention are advantageous in that they allow the washing, skin-care and possibly disinfection of the skin by applying the compositions after washing without requiring an intermediate step of actively drying the hands by a towel or a mechanical/electric means. The present invention is applicable in particular in environments requiring high hygiene levels and has economic advantages compared to current sanitary solutions in this field which require either the use of towels or electric ventilators/fans for drying the skin after washing.

### Background of the invention

In the field of hand drying technology, various solutions have been developed and placed on the sanitary products market. The most well-known method is the drying of hands, body and hairs using cotton towels. They show a high level of water absorbency and can be reused for years through regular appropriate laundry. Using the cotton towel basis various towel dispensing systems have been developed. For example, US5377427A discloses a device combining a rotating towel support with warm air blower. The big disadvantage of the cotton towel drying solution is the required laundry process accompanied with continuous energy costs, water consumption and resulting negative environmental impact due to actual washing machine technology and transportation of dirt and clean towels. This negative impact is not compensated by the re-usage possibility. Additionally, the used wet and dirt cotton towels are usually stored at ambient temperature for hours or even days. This humid and warm environment supports bacterial and fungal growth.

Faced with usual concerns accompanied with cotton towels the sanitary industry developed towels made of paper. Such paper sheets are usually dropped into a bin after the first usage. Thus, due to the humid and warm surrounding present in the bin. There are similar hygienic issues as for cotton towels. Furthermore, the paper towels are made of wood and the even if the towels are made of recycled paper, the production process requires a huge amount of water and energy. Additionally, the garbage bin has to be emptied regularly to avoid littering and avoidance of unhygienic circumstances in the sanitary area. DE 20 2016 003 553 U1 describes a dispenser for a paper towel-glove combination which allows a hygienic leave of sanitary rooms. Due to the included hand protection the door handle can be touched avoiding contamination and/or transfer of bacteria, viruses and fungi. US 5048589A discloses a paper towel showing enhanced absorbent capacity, absorbent rate, softness and strength compared to prior art paper towels having similar basis weight. In US3785523A a hand drying equipment is described which combines a vertical stack of C-folded paper towels with a hot air blower.

Another development of the hand drying process in sanitary areas was evaporation using a warm air stream or blowing the water off the hands using a strong air stream or a mixture of both. EP 2684497 A2 discloses a device using air stream and radiant warmers for drying of hands. US4876435A describes a device for drying hands using hot air directed over, under and around the hands. In US7039301B1 a dryer is described using high speed flow of heated air. The forceful air jet blows off most of the loose water on the hands and improves the evaporation removal of the remaining water film. This device is further developed in US20090119942A1 which describes a high-speed blower equipped with particularly engineered nozzles and additionally integrated electrical discharges (electrons, ions). This system enables fast (< 30 seconds), comfortable (feeling of warmth, residual water amount of 0.20 g or less) drying of hands and the device's noise is reduced (< 95 dB). US6038786A discloses a blower generating warm air flow which is pulsated or modulated in order to break down the boundary layer disposed on the hand surface resulting in short drying time. US3744149A describes a device which dries hands by application of heated air and flakes of absorbent material in a drying shell. The flakes are blown from the hands into a waste bag.

These air blowing systems exhibit similar hygienic problems like cotton towels and paper sheets. In addition, the applied high air speeds support the extensive spreading of such bacteria, viruses and fungi in the sanitary area. Studies showed that using UV light or other antibacterial equipment may reduce the bacteria load. In US3667134A an electrical hand dryer is disclosed which uses heated air, includes moisture absorbing rods for drying the air and includes germicidal lamps or HEPA filters for cleaning the air. However, dependent on the type of bacteria and viruses which can be found in the air systems or on the hands itself, a higher risk of infection is present (refer to P.T. Kimmit, K.F. Redway, Journal of Applied Microbiology, 120, 478-486, 2015).

Various electric systems have been developed in order to combine drying and disinfection of hands and/or the sanitary area. US5074322A describes a hand dryer which sprays antiseptic solution on the user's hand and dries them with hot air. DE 20 2005 005 894 U1 describes a device applying heated air for drying and ozone used for disinfection and as deodorant for the sanitary area.

US20080008865A1 discloses a disposable paper towel with a transferable lotion composition comprising an emollient and anti-microbial agent immobilized on the cellulosic web in a semi-solid or solid form. The lotion composition is transferable upon contact with water or application of body heat.

EP 0 604 848 A2 describes a quick-drying, gel-type disinfectant composition comprising a disinfectant in an alcohol solution and a thickening agent consisting of a combination of 0.05 to 2.0 wt.-% of carboxyvinyl polymer and a water-soluble, high molecular cellulose compound contained in an amount of 0.1 to 2.5 wt.-%, provided that the total weight of both components is not larger than 3.0 wt.-%. This application can be well spread onto the hands avoiding scale-like residue or unpleasant stickness before or after drying.

US 6,627,207 B1 discloses a water-based, quick-drying, gel-type disinfectant composition comprising anti-microbial ingredients, 0-30 % of an alcohol, a thickening agent, an emulsifier, and water. The thickening agent is typically selected from acrylic acid copolymers, methacrylic acid copolymers, sodium carboxycellulose, hydroxyethylcelluslose and hydroxypropylcellulose. A preferred embodiment comprises 0.05-5.0 % of fatty alcohol polyglycol ether as an emulsifier, 0.5-5.0 % of glycerin, 0.01-2.0 % of one of more additives including 0.05-1.0 % of a basic compound and substantially the balance of water. According to the inventor, said composition shows a drying time of about 45 sec.

In US 5,997,889 A, a composition for hand and body cream for the treatment and relief of skin ailments such as itching, dryness, eczema, psoriasis and rashes, and more particularly, to hand and body cream compositions comprising admixtures of naturally occurring ingredients is described. The mixture contains 25 - 35 v% of almond oil, 18 - 28 v% of cacao butter, 18 - 28 v% of jojoba oil, 5 - 10 v% of vitamin E, 5 - 10 v% of a beeswax derivative, 2 - 6 v% soybean flakes, 2 - 6 v% of beeswax and 0.2 - 1 v% of vitamin A.

FR 2 471 775 A1 discloses cosmetic oils containing a mixture of at least two vegetable oils which are at least 20 - 45 wt.-% of jojoba oil and 25 - 40 wt.-% of sunflower oil, and which further contains at least 20 - 40 wt.-% of one unsaponifiable fraction previously extracted and/or pistachio oil. This cosmetic oil may be added in the amount of 10 - 50 wt.-% to a cosmetic composition like body milk, care cream or sun cream which may contain small amounts of Carbopol (about 0.4 wt.-%) as thickening agent.

DE 4 425 268 A1 describes finely dispersed, emulsifier-free cosmetic or dermatologic oil-in-water type preparations containing oils (covalent oils, fats and/or waxes) as main component, an aqueous phase, thickeners such as acrylic acid polymers (like carbomers or carbopol), polysaccharides and their alkyl ethers and optionally conventional cosmetic or dermatologic auxiliary substances, additives and/or active substances. Such preparations contain 0.5 - 50 wt.-% of one or more non-polar oils preferably selected from the group vaseline, paraffin oil and/or polyolefins, and 0.005 - 10 wt.-% thickeners, auxiliary substances, additives or active ingredients and water.

Various cosmetic companies sell quickly absorbing oils and hand creams, like e.g. Neutrogena® Norwegian Formula Body Oil, Netrogena® Norwegian Formula Fast Absorbing Hand Cream, Dove® DermaSpa Goodness Body Oil, containing natural/synthetic oils, dermatologic additives and thickeners such as acrylate crosspolymer or xanthan gum. These oils and creams are quickly absorbed by dry hands, but they are not drying wet skin in an appropriate period of time.

The quick drying inventions mentioned above and the current cosmetic skin care products do not exhibit exceptional drying properties which would enable them to be supplements to current hand skin drying solutions in the sanitary section or healthcare area. This is also true for gels which are mostly used for disinfection of skin applied after drying washed skin with cotton/paper towels or by a warm air stream.

### Objects of the present invention

As described above, common drying solutions applied in sanitary areas require high consumption of water and/or energy and may generate high costs for equipment cleaning or garbage bin emptying. It is, therefore, an object of the present invention to provide a drying solution which is more hygienic and more cost efficient than current solutions, and which also provides care to the skin. Additionally, the time of drying skin after washing with water and soap should be acceptable for ordinary applicants (between 15 and 60 seconds).

In order to obtain a composition such as a semisolid wax, a cream, a lotion or a gel which is feasible to reach the set targets, the final combination of substances should preferably fulfill various ecologic requirements. The application of the composition should not be made with the use of paper or cotton sheets or towels to reduce the ecological footprint generated during production as well as through the cleaning process of cotton towels.

Energy consumption of the invention should be reduced to a minimum compared to current warm air dryers or towel dispensers. The desired solution should be via a common and/or modified mechanical or low energy dispenser.

In order to obtain the health protection requirements for sanitary or other sensitive areas, e.g. in hospitals, pharma industry, etc., the invention should not present a source for bacteria, fungi or virus growth and should be modifiable e.g., but not limited to, showing highly disinfecting properties in certain embodiment, when required.

Indeed, the invention should not be irritating to skin. Thus, dermatology tests need to show that the composition which should normally be in the form of a semisolid wax, cream, lotion or gel is suitable for daily hygienic application, in particular for drying hands after washing them with water and soap.

In summary, the current invention should solve various technical problems resulting from the desired skin care and drying properties of such compositions, optionally supplemented by disinfection properties in certain embodiments, when required.

To solve the object(s) of the invention, the present invention provides:
1. A skin-care and drying composition, comprising:
   a. at least one oil component which is liquid at 25 °C, in a total content of at least 3 wt.-%,
   b. at least one gelling agent selected from the group consisting of homopolymers of acrylic acids, copolymers of acrylic acids, cellulose derivatives and polysaccharides, in a total content of more than 5 to 50 wt.-%,
   c. at least one emulsifier,
   d. at least one fat or wax component which is solid at 25 °C,
   e. at least one moisturizer and
   f. 30 wt.-% or less of water, wherein all contents relate to the total skin-care drying composition.
2. The skin-care drying composition according to 1, comprising:
   a. 10 to 60 wt.-% of said at least one oil component,
   b. 10 to 35 wt.-% of said at least one gelling agent,
   c. 5 to 30 wt.-% of said at least one emulsifier,
   d. 5 to 25 wt.-% of said at least one fat or wax component,
   e. 5 to 35 wt.-% of said at least one moisturizer, and
   f. less than 1 wt.-% of water.
3. The skin-care drying composition according to 2, comprising:
   a. 20 to 40 wt.-%, preferably 25 to 35 wt.-%, of said at least one oil component
   b. 17 to 30 wt.-%, preferably 20 to 25 wt.-%, of said at least one gelling agent,
   c. 5 to 20 wt.-%, preferably 7 to 15 wt.-% of said at least one emulsifier,
   d. 10 to 20 wt.-%, preferably 12 to 18 wt.-%, of said at least one fat or wax component, and
   e. 7 to 25 wt.-%, preferably 10 to 20 wt.-% of said at least one moisturizer.
4. The skin-care drying composition according to any one of the preceding points, further comprising:
   g. at least one cosmetically acceptable alcohol.
5. The skin-care drying composition according to 4, comprising:
   a. 7 to 20 wt.-% of said at least one oil component,
   b. 6 to 15 wt.-% of said at least one gelling agent,
   c. 2 to 10 wt.-% of said at least one emulsifier,
   d. 3 to 10 wt.-% of said at least one fat or wax component,
   e. 2 to 10 wt.-% of said at least one moisturizer, and
   g. 5 to 65 wt.-% of said at least one alcohol, wherein said alcohol is a monohydric alcohol.
6. The skin-care drying composition according to any one of the preceding points, wherein said oil component a. is one or more selected from the group consisting of plant oils and fatty acid ester compounds.
7. The skin-care drying composition according to any one of the preceding points, wherein said gelling agent b. is one or more selected from the group consisting of polysaccharides and homopolymers of acrylic acids.
8. The skin-care drying composition according to any one of the preceding points, wherein said emulsifier c. is one or more selected from the group consisting of mono- or difatty acid esters of glycerin, polyglyceryl fatty acid esters, lecithins and modified lecithins.
9. The skin-care drying composition according to any one of the preceding points, wherein said fat or wax component d. is at least one selected from the group consisting of fatty alcohols and triglycerides of fatty acids.
10. The skin-care drying composition according to any one of the preceding points, additionally comprising one or more selected from the group consisting of an antibacterial agent, an antiviral agent and an antifungal agent.
11. The skin-care drying composition according to any one of the preceding points, wherein the composition additionally comprises one or more selected from the group consisting of coenzymes, vitamins, provitamins, pH regulating substances, fragrances, preservatives and UV protectors.
12. The skin-care drying composition according to any one of the preceding points, wherein the composition comprises:
   a. one or more oil component selected from the group consisting of plant oils, such as jojoba oil, grape seed oil, apricot kernel oil, sesame oil, coconut oil, babassu oil, olive oil, avocado oil, almond oil, argan oil, aloe vera oil, candula oil, evening primrose oil, fatty acid esters, such as isoamyl laurate and isopropyl myristate,
   b. one or more gelling agent selected from the group consisting of xanthan gum, guar gum, agar-agar, locus bean gum, and carbomers,
   c. one or more emulsifier selected from the group consisting of lecithin, modified lecithins, glyceryl monostearate and glyceryl distearate,
   d. one or more fat or wax component selected from the group consisting of lanolin, cetyl alcohol, shea butter, cupuaca butter, cacao butter, lama cream and mango butter,
   e. one or more moisturizers selected from the group consisting of polyols such as glycerol, 1,2-propanediol, sorbitol, hyaluronic acid and ceramides, and urea, - optionally one or more alcohol selected from the group consisting of ethanol, propanol, isopropanol and 1-tetradecanol.
13. The skin-care and drying composition according to any one of the preceding points, which is in the form of a wax, semisolid wax, a cream, a lotion or a gel.
14. Use the skin-care and drying composition according to any one of points 1 to 13 for drying the skin or drying and disinfecting the skin.
15. A non-medical method of washing and drying hands, comprising:
   - washing the hands,
   - applying the composition according any one of points 1 to 13 to the wet hands, and
   - drying the composition by exposure to air.
16. The method according to 15, which does not comprise a step of drying the hands with a towel and/or a step of drying the hands using a device for generating a forced air stream.
17. The method according to 15 or 16, comprising washing the hands with a detergent-containing composition, normally rinsing the hands with water, removing excess liquid from the hands by shaking the hands, applying the composition to the wet hands followed by rubbing the hands to spread the composition, and exposing the hands to air, resulting in dried (residual water amount of 0.20 g or less) and cared hands in less than 60 seconds.
18. The method according to 17, wherein the process does not comprise touching any object when removing the excess liquid from the hands and exposing the hands to air.

### Detailed description of the invention

The present invention provides a combination of cosmetic components which is capable of drying and caring skin, and optionally disinfecting the skin. When referring to the "skin" in the present invention, generally the skin of hands is meant. However, the term skin should not be restricted to mean only the skin of hands but the composition and the method of the present invention can be applied to skin of other parts of the human body as well. However, the composition of the present invention is typically applied to the skin of hands after washing, and the application of the composition of the present invention can replace the use of towels and apparatus for generating a forced air stream to dry the hands. At the same time, the composition of the present invention has a skin-caring effect. Hence, a subsequent step of applying a cream or the like, which is often the case when hands are washed frequently, is not necessary.

In the present description, weight ranges apply to the total composition, respectively, unless stated otherwise. Further, contents of components indicated in the present invention relate to the active content of said component unless stated otherwise.

Surprisingly, it was found by the inventors that the presence of at least one gelling agent selected from the group consisting of homopolymers of acrylic acids, copolymers of acrylic acids, cellulose derivatives and polysaccharides, in a high amount, i.e. in a total content of more than 5 to 50 wt.-%, in combination with further cosmetic components and with a low water content allows to provide a composition which quickly dries wet hands after washing and at the same time provides a good level of skin-care.

The presence of gelling agent in a high amount is an essential aspect of the present invention. The gelling agent in the present invention is to be understood as a compound which forms a gel when contacted with water. The gelling agent b. is at least one selected from the group consisting of homopolymers of acrylic acids, copolymers of acrylic acids, cellulose derivatives and polysaccharides. More preferably, the gelling agent is at least one selected from the group consisting of homopolymers of acrylic acids and polysaccharides, and more preferably at least one homopolymer of an acrylic acid. Homopolymers of acrylic acids are commercially available as Carbomer (INCI) or Carbopol. Preferred are carbomers having a viscosity in the range of 40000 to 60000 mPa·s, the viscosity being a relative viscosity determined at a concentration of 0.5 wt.-%, pH 7.3-7.8 at 25 °C with a Brookfield rotary viscometer, spindle #7. For the purposes of the present invention, Carbopol 980 is particularly suitable, which is available for example as Carbomer 980/Carbopol 980 Powder by OTC Pharma. Other preferred gelling agents in the present invention which can be used in addition or alternatively, are cellulose derivatives such as carboxy methylcellulose and hydroxyethyl cellulose, or other polysaccharides such as xanthan gum, guar gum, agar-agar, locus bean gum.

The compositions of the present invention contain 30 wt.-% or less of water, preferably 27 wt.-% or less water, more preferably 25 wt.-% or less, further preferably 20 wt.-%, or less of water. In compositions containing water, the lower limit of the water content is preferably 3 wt.-% or more, more preferably 4 wt.-% or more or 5 wt.-% or more. Among these, compositions having a rather high water-content can have a gel-like appearance or form. Compositions of the present invention having a water-content in the lower end range tend to have a more solid or wax-like appearance or form. Generally, the composition of the present invention can preferably be provided in the form of a solid wax, semi-solid wax, cream, lotion or gel. Compositions at the more solid end may require a longer drying time but can provide more nurturing effects to the skin due to contained oil, fat and and/or wax components. More gel-like composition having generally a lower viscosity may result in shorter drying times of the hands.

In preferred embodiments of the present invention, the compositions of the present invention are essentially water-free; for example, having a total water content of less than 3 wt.-%, more preferably less than 2 wt.-%, more preferably less than 1 wt.-% or no water. The water content, as understood in the present invention, relates to the total composition and includes water introduced into the composition via added components. In the present invention, the water content can be determined by the Karl Fischer method. Such essentially water-free compositions of the present invention have more of a wax-like form and appearance. Further, such inventive, essentially water-free compositions or inventive compositions containing no water, preferably have the following contents of other components: 10 to 60 wt.-%, preferably 20 to 40 wt.-%, more preferably 25 to 35 wt.-% total of the at least one oil component a.; 15 to 35 wt.-%, more preferably 17 to 30 wt.-%, more preferably 20 to 25 wt.-% total of the at least one gelling agent b.; 5 to 30 wt.-%, more preferably 7 to 20 wt.-%, more preferably 8 to 15 wt.-% total of the at least one emulsifier; 5 to 25 wt.-%, more preferably 10 to 20 wt.-%, more preferably 12 to 18 wt.-% total of the at least one fat or wax component; and 5 to 35 wt.-%, more preferably 7 to 25 wt.-%, more preferably 10 to 20 wt.-% of the at least one moisturizer e.

In preferred embodiments of the present invention, the compositions contain at least one cosmetically acceptable alcohol, preferably a monohydric alcohol which is more preferably selected from the group consisting of ethanol, propanol, isopropanol and 1-tetradecanol. Such monohydric alcohols can improve the disinfecting properties of the compositions of the present invention. The alcohol can preferably be contained in a total amount of 5 to 65 wt.-%, more preferably 10 to 65 wt.-%, more preferably 20 to 60 wt.-% even more preferably 30 to 55 wt.-%, further preferably 40 to 50 wt.-%.

When the skin-care and drying composition of the present invention comprises a cosmetically acceptable alcohol, the composition preferably comprises:
a. 7 to 20 wt.-%, preferably 8 to 15 wt.-%, of said at least one oil component,
b. 6 to 15 wt.-%, preferably 6 to 10 wt.-%, of said at least one gelling agent,
c. 2 to 10 wt.-%, preferably 2 to 5 wt.-%, of said at least one emulsifier,
d. 3 to 10 wt.-%, preferably 4 to 8 wt.-% of said at least one fat or wax component,
e. 2 to 10 wt.-%, preferably 3 to 8 wt.-%, of said at least one moisturizer, and
g. 5 to 65 wt.-% of said at least one alcohol, wherein said alcohol is a monohydric alcohol.

The used substances used in the composition of the present invention may stem from natural or organic sources or may be synthetic though they are preferably natural or organic substances or are isolated or derived therefrom.

The compositions of the present invention contain at least one oil component a. which is liquid at ambient temperature (25 °C), in a total content of at least 3 wt.-%. The total content of the oil component in the present invention is preferably at least 5 wt. %, more preferably at least 7 wt.-%, further preferably at least 8 wt.-% or at least 10 wt.-%, with respect to the total composition. The total content of the oil component is preferably 60 wt.-% or less, more preferably 50 wt.-% or less, 40 wt.-% or less, further preferably 35 wt.-% or less, in case the composition contains 3 wt.-% or less of water. In case the composition of the present invention contains an alcohol f., the total content of the oil component is preferably 30 wt.-% or less, more preferably 20 wt.-% or less, further preferably 15 wt.-% or less or 10 wt.% or less. The type of oil component is not particularly restricted and cosmetically acceptable oil components are known to the skilled person. The oil component can comprise volatile or non-volatile oils of vegetable, mineral or synthetic origin, and their mixtures.

Examples for the oil components are oils of animal origin, oils of vegetable origin, such as liquid triglycerides of fatty acids comprising from 4 to 10 carbon atoms, such as triglycerides of heptanoic acid or octanoic acid, or also, for example, jojoba oil, grape seed oil, apricot kernel oil, sesame oil, coconut oil, babassu oil, olive oil, avocado oil, almond oil, argan oil, aloe vera oil, candula oil, evening primrose oil, sunflower oil, maize oil, soybean oil, cucumber oil, hazelnut oil, macadamia oil, arara oil, coriander oil, triglycerides of caprylic/capric acids, jojoba oil or shea butter oil, medium-chain triglycerides (MTC) and mixtures thereof, synthetic esters and ethers, in particular of fatty acids, such as oils of formulae R¹COOR² and R¹OR² in which R¹ represents the residue of a fatty acid or of a fatty alcohol comprising from 8 to 29 carbon atoms and R² represents a branched or unbranched hydrocarbon chain comprising from 3 to 30 carbon atoms, such as, for example, 2-octyldodecyl stearate, 2-octyldodecyl erucate or isostearyl isostearate; hydroxylated esters, such as isostearyl lactate, octyl hydroxystearate, octyldodecyl hydroxystearate, diisostearyl malate, triisocetyl citrate or heptanoates, octanoates or decanoates of fatty alcohols; polyol esters, such as propylene glycol dioctanoate, neopentyl glycol diheptanoate and diethylene glycol diisononanoate; and pentaerythritol esters, such as pentaerythrityl tetraisostearate; linear or branched hydrocarbons of mineral or synthetic origin, such as volatile or non-volatile liquid paraffins and their derivatives, liquid petrolatum, polydecenes, isohexadecane, isododecane or hydrogenated polyisobutene; silicone oils, such as volatile or non-volatile polymethylsiloxanes (PDMSs) comprising a linear or cyclic silicone chain which are liquid or pasty at ambient temperature, in particular volatile silicone oils, especially cyclopolydimethylsiloxanes (cyclomethicones), such as cyclohexadimethylsiloxane and cyclopentadimethylsiloxane; polydimethylsiloxanes comprising pendant alkyl, alkoxy or phenyl groups or alkyl, alkoxy or phenyl groups at the end of the silicone chain, which groups have from 2 to 24 carbon atoms; or phenylated silicones, such as phenyl trimethicones, phenyl dimethicones, phenyl(trimethylsiloxy)diphenylsiloxanes, diphenyl dimethicones, diphenyl(methyldiphenyl)trisiloxanes, (2-phenylethyl)trimethylsiloxysilicates and polymethylphenylsiloxanes; and their mixtures.

In the present invention, oils of vegetable origin such as jojoba oil, grape seed oil, apricot kernel oil, sesame oil, coconut oil, babassu oil, olive oil, avocado oil, almond oil, argan oil, aloe vera oil, candula oil, evening primrose oil, sunflower oil, maize oil, soybean oil, cucumber oil, hazelnut oil, macadamia oil, arara oil, coriander oil, triglycerides of caprylic/capric acids, jojoba oil or shea butter oil are preferred. Among these, plant oils, such as jojoba oil, grape seed oil, apricot kernel oil, sesame oil, coconut oil, babassu oil, olive oil, avocado oil, almond oil, argan oil, aloe vera oil, candula oil, evening primrose oil, isopropyl myristate and combinations thereof are particularly preferred.

As a further component, the compositions of the present invention contain at least one emulsifier (component (c.). The emulsifier or emulsifying agent is not particularly limited and nonionic or ionic surfactants, particularly anionic and amphoteric or zwitterionic surfactants, may be used in the present invention. The skilled person can select from a wide range of known cosmetically accepted emulsifying agents.

Suitable emulsifying agents are for example, glycerin fatty acid esters, betaine-type surfactants, phosphatidyl choline-type surfactants and mixtures of these.

The glycerin fatty acid ester is an ester of glycerin and fatty acid(s). It is preferable that the glycerin fatty acid ester be a monoester, wherein one molecule of a fatty acid is ester bonded to any hydroxyl group of glycerin. Examples of the fatty acids include straight-chain fatty acids, branched-chain fatty acids, saturated fatty acids, unsaturated fatty acids and hydroxy fatty acids. It is preferable that the glycerin fatty acid ester is non polyoxyalkylenated.

Examples of the glycerin mono fatty acid esters include glyceryl caprylate, glyceryl caprate, glyceryl laurate, glyceryl myristate, glyceryl stearate, glyceryl linoleate, glyceryl oleate, glyceryl isostearate, glyceryl behenate, glyceryl erucate, glyceryl cocofatty acid ester, glyceryl ricinoleate, glyceryl hydroxystearate, wheat germ oil fatty acid monoglyceride, safflower oil fatty acid monoglyceride, hydrogenated soybean fatty acid monoglyceride, saturated fatty acid monoglyceride, cotton seed oil fatty acid monoglyceride, tallow fatty acid monoglyceride and lanolin fatty acid monoglyceride.

The glycerin fatty acid ester may preferably be a monoester of glycerin and C8-C18 fatty acid, preferably C8-C14 fatty acid, and more preferably C8-C12 fatty acid. Thus, the glycerin fatty acid ester may preferably be selected from the group consisting of glyceryl caprylate, glyceryl caprate, glyceryl laurate, glyceryl myristate, glyceryl stearate, glyceryl isostearate and mixtures thereof.

The amphoteric or zwitterionic surfactants can be, for example, amine derivatives such as aliphatic secondary or tertiary amine, and optionally quaternized amine derivatives, in which the aliphatic radical is a linear or branched chain comprising 8 to 22 carbon atoms and containing at least one water-solubilizing anionic group (for example, carboxylate, sulfonate, sulfate, phosphate or phosphonate). The amphoteric or zwitterionic surfactants can further preferably be selected from phosphatidylcholines.

Non-limiting examples for betaines that may be mentioned include the compounds classified in the CTFA dictionary, 9th edition, 2002, under the names cocobetaine, laurylbetaine, cetylbetaine, coco/oleamidopropylbetaine, cocamidopropylbetaine, palmitamidopropylbetaine, stearamidopropylbetaine, cocamidoethylbetaine, cocamidopropylhydroxysultaine, oleamidopropylhydroxysultaine, cocohydroxysultaine, laurylhydroxysultaine, and cocosultaine, alone or as mixtures.

Preferred emulsifying agents are for example, but not limited to, selected from lecithin, modified lecithins, glyceryl monostearate and glyceryl distearate, (commercially available for example as fluid lecithin, lysolecithin), methyl glucose sesquistearate (INCI) (Emulsan), glyceryl monostearate, glyceryl distearate, Polyglyceryl 3-Palmitate (INCI) (Softfeel). Commercially available blends containing surfactants besides other components may also be used, such as commercially available Rimulgan which contains castor oil and about 25 wt.-% of ionic oleic acids.

The composition of the present invention may contain a total of 0.5 to 30 wt.-% of the emulsifying agents, preferably 1 to 20 wt.-%, more preferably 2 to 15 or 3 to 15 wt.-% or 3 to 12 wt.-%. In case of the compositions of the present invention containing at least one alcohol in a higher amount, the total content of emulsifying agents is preferably at the lower end, such as preferably 0,5 to 10 wt.-%, more preferably 1 to 5 wt.-%. The contents refer to the content of active surfactant components in the composition of the present invention.

The compositions of the present invention further contain at least one fat or wax component (component d.). In the present invention, a fat or wax component is to be understood as a component which is solid at 25 °C. Such compounds are mainly included as consistency enhancers or for increasing the viscosity of the compositions of the present invention but also contribute to the skin-caring effect of the composition.

The term "fat or wax component" in the present invention relates to a a lipophilic compound, which is solid at room temperature (25° C), with a reversible solid/liquid change of state, having a melting point of normally greater than or equal to 30° C. In particular, the waxes may have a melting point of greater than or equal to 30° C or greater than or equal to 45° C.

Examples of the fat or wax component used in the present invention include a natural wax but a synthetic wax may also be used. Examples of the natural wax include a petroleum wax, a plant wax, and an animal wax. Examples of the petroleum wax include a paraffin wax, a microcrystalline wax, and a petrolatum. Examples of the plant wax include rice wax, carnauba wax, candelilla wax, ouricury wax, Japan wax, cocoa butter, shea butter, cupuaca butter, cacao butter, lama cream, mango butter cork fibre wax and sugarcane wax. Examples of the animal wax include lanolin wax, lanolin derivatives and beeswax. Examples of the synthetic wax include a synthetic hydrocarbon wax and a modified wax.

Examples of the synthetic hydrocarbon wax include polyethylene wax, polypropylene wax, and Fischer-Tropsch wax. Examples of the modified wax include a paraffin wax derivative, a montan wax derivative, and a microcrystalline wax derivative. Examples of the polyethylene wax include an ethylene homopolymer and an ethylene-alpha-olefin copolymer. Alternatively, the wax may be obtained by thermal decomposition of the copolymer. Examples of the alpha-olefin include an alpha-olefin having 3 to 12 carbon atoms such as propylene, 1-butene, 1-pentene, 1-hexene, 4-methyl-1-pentene, and 1-octene.

The Fischer-Tropsch wax is a synthetic hydrocarbon wax mainly comprising linear hydrocarbons, which is obtained by reacting water gas containing carbon monoxide and hydrogen as main components under normal pressure at 170 to 250° C. using a catalyst such as cobalt, nickel, or iron. The Fischer-Tropsch wax is characterized in comprising hydrocarbons containing odd and even numbers of carbon atoms, namely comprising both hydrocarbons containing odd numbers of carbon atoms and hydrocarbons containing even numbers of carbon atoms.

The fat or wax component in the present invention is preferably a compound selected from the group consisting of fatty alcohols and triglycerides of fatty acids. Specific preferred examples in the present invention are lanolin, cetyl alcohol, shea butter, cupuaca butter, cacao butter, lama cream and/or mango butter.

The at least one fat or wax component is contained in the composition of the present invention having a low water content or containing no water preferably in a total of 5 to 25 wt.-%, more preferably 10 to 20 wt.-%, more preferably 12 to 18 wt.-%. In embodiments of the present invention comprising a cosmetically acceptable alcohol g., the at least one fat or wax component is preferably contained in a total content of 3 to 10 wt.-%, preferably 4 to 8 wt.-%.

The composition of the present invention further contains at least one moisturizer f. The moisturizer is preferably a polyol and is further preferably selected from a polyol selected from the group consisting of glycerol, 1,2-propanediol, sorbitol, hyaluronic acid and ceramides, and urea. In particularly preferred embodiments, the moisturizer is glycerol.

The moisturizer f. can be contained in the composition of the present invention in a total content of preferably 2 or more wt.-%, more preferably 3 or more wt.-%, further preferably 5 or more wt.-% or 7 or more wt.-% or 10 or more wt.-%. Further, the moisturizer f. is preferably contained in a total content of 35 or less wt.-%, more preferably 25 or less wt.-%, more preferably 20 wt.-% or less, further preferably 15 wt.-% or less or 10 wt.% or less. In compositions of the present invention which are essentially water-free (3 wt.-% or less of water), the moisturizer f. is preferably contained in an amount of 5 to 35 wt.-%, more preferably 7 to 25 wt.-%, more preferably 10 to 20 wt.-% of the at least one moisturizer f. In embodiments of the present invention comprising a cosmetically acceptable alcohol g., the at least one moisturizer is preferably contained in a total content of 2 to 10 wt.-%, preferably 3 to 8 wt.-%.

In preferred embodiments of the present invention, the composition contains at least one disinfecting agent selected from an antibacterial agent, an antiviral agent and/or antifungal agent. Such compositions are preferred in that they improve the disinfection properties and are therefore particularly suitable for application in healthcare environments such as hospitals, medical practices, hospitals etc. Examples of disinfecting agents which can be added are mecetroniumetilsulfat, dodecyldimonium chloride or others.

The composition of the present invention can contain further additives. For example, vitamins, pH regulating substances, UV protectors, preservatives, fragrances, protein extracts, coenzymes, vitamins, provitamins, plant extracts etc. may be added. Examples of specific substances which can be contained in composition of the present invention are avocadin, aloe vera gel, candula extract, co-enzyme Q10, d-panthenol, allantoin, tocopherol, collagen, sodium lactate, silk protein, vitamin C, vitamin E.

For conservation, potassium sorbate, grapefruit seed extract, Biokons (INCI: Propanediol, Phenethyl Alcohol, Undecyl Alcohol, Tocopherol), lactic acid, Rokonsal (INCI: Benzyl Alcohol, Glycerin, Benzoic Acid, Sorbic Acid), Dermosoft 1388 (INCI: Glycerin, Aqua, Sodium Levulinate, Sodium Anisate), or others may be used. In case such additives contain substances belonging to either of components a. to f. described above, these would have to be assigned to the content of a. to f.

Examples of fragrances which can be used in the present invention are litsea oil, orange oil, lavender oil, vanilla oil, violet oil or others.

The compounds contained in the composition of the present invention are preferably selected so that the composition of the present invention passes the skin compatibility tests like ISO 10993.

The present invention also relates to the use of the composition of the present invention for drying the skin or drying and disinfecting the skin, preferably the skin of hands.

The present invention further relates to a non-medical method of washing and drying the skin, normally the skin of hands, comprising washing the hands, usually followed by rinsing with water, such as tap water, applying the composition according to the invention to the wet hands, and drying the composition by exposure to air.

The method of washing and drying the skin, normally the skin of hands, more specifically comprises washing the hands, applying the composition of the present invention to the wet hands (or skin in general) after washing, usually followed by rinsing with (tap) water, spreading the composition by rubbing the hands and drying the mixture, formed of water that remained on the hands water and the composition of the present invention, by exposure to air. Washing the hands/skin can be carried out using a standard aqueous detergent composition such a soap, liquid soap etc., or an aqueous disinfectant detergent. After washing the hands with a detergent, usually followed by rinsing with water, excess water is removed from the hands, preferably by shaking the hands, followed by applying the composition of the present invention, preferably without contacting any object such as a towel and without exposing the hands to a forced air flow.

The exposure of the hands/skin to air after having applied the composition of present invention is preferably carried out for less than 60 seconds, preferably 45 seconds or less or 30 seconds or less. After such a period of time, the composition of the present invention has sufficiently dried and/or has been absorbed by the skin while at the same time having imparted skin-care to the washed hands or skin. After this, objects or persons can be touched.

Preferably, the invention described above can be used at home and/or more preferably in public sanitary areas where the spread of bacteria and viruses through the high visitor traffic is usually high. By using the described invention, the risk of infection can be reduced. Additionally, the application of the invention leads to cost savings due to avoidance of cotton towel laundry or paper towel disposal or the need of electric energy for air stream blowers which also lowers the economic footprint.

The present invention is also applicable in health care companies like e.g. hospital, pharma industry, medical industry, etc., where a particular focus is on minimization of potential infective risks like e.g. in clean rooms or intensive care units or operation rooms (medicine).

### Examples

### Comparative Example 1: Commercial paper towels

Commercial paper towels *"ULTRA SUPER SOFT Interfold 3 layers"* from Kleenex®.

### Comparative Example 2: Commercial air blower

Commercial air blower *"Dyson dB Hand Dryer* - *Model AB 14".*

### Example 1: Wax

In a glass beaker 0.20 g jojoba oil (Dragonspice Naturwaren), 0.20 g neutral oil (Dragonspice Naturwaren), 0.30 g Dermofeel® Sensolv (Evonik Dr Straetmans GmbH) and 0.30 g fluid lecithin (Dragonspice Naturwaren) were mixed. Then a solution of 0.50 g shea butter (Dragonspice Naturwaren) and 0.3 g coconut oil (aliacura) was added. Finally, 0.70 g Carbomer (Carbomer 980/Carbopol 980 Pulver, Euro OTC Pharma), 0.50 g glycerin (Dragonspice Naturwaren) and 0.05 g litsea oil (Dragonspice Naturwaren) were added to the oily solution to result in a semisolid yellowish wax.

### Example 2: Gel

In a glass beaker 0.16 g jojoba oil Bio (Dragonspice Naturwaren), 0.16 g neutral oil (Dragonspice Naturwaren), 0.25 g Dermofeel® Sensolv (Evonik Dr Straetmans GmbH), 0.25 g fluid lecithin (Dragonspice Naturwaren), 0.42 g glycerin (Dragonspice Naturwaren) and 0.05 g litsea oil (Dragonspice Naturwaren) were mixed. Then a solution of 0.42 g shea butter and 0.25 g coconut oil was added. Finally, a mixture of 0.6 g Carbomer (Carbomer 980/Carbopol 980 Pulver, Euro OTC Pharma) and 6.0 g Isopropanol (70% V/V, Otto Fischar GmbH & Co. KG) were added to oily solution to result in a pale, yellow gel.

### Test Conditions:

The test persons washed their hands with soap and water. After rinsing with water, that they shook their hands twice in order to remove the excess of water. Then the example compositions prepared as described above were applied which included rubbing of hands to spread the compositions of the present invention evenly. An amount of 0.6 g of the wax prepared in Example 1, and an amount of 1.3g of the gel prepared in Example 2 were used to this end. After 15, 30, 45, and 60 seconds, one hand was pressed on a piece of paper (DIN/A4, 80 g/m²) for about 2 seconds and then moved from a horizontal to a vertical position. The hands were defined to be "dry" as soon as the lifted piece of paper did not stick to the hand in the vertical position.

For comparison, the test persons dried their hands with a paper towels (*"ULTRA SUPER SOFT Interfold 3 layers"* from Kleenex*®*), or using an air blower (*"Dyson dB Hand Dryer* - *Model AB* 14"), followed by the same aforementioned dryness test.

The "Drying Time" in Table 1 is defined as the time period starting with application of the drying agent (e.g. paper towel, air blower, wax, gel) until the point in time when the hands are "dry" as defined above.

**Tab. 1: Drying times of examples and comparative examples (average of at least ten tests).**

| | Drying Time | | | |
|---|---|---|---|---|
| | 15 s | 30 s | 45 s | 60 s |
| Comparative Example 1 (paper towel) | X | | | |
| Comparative Example 2 (air blower) | | X | | |
| Example 1 (wax) | | | X | |
| Example 2 (gel) | | X | | |

## Claims

1. A skin-care and drying composition, comprising:
a. at least one oil component which is liquid at 25 °C, in a total content of at least 3 wt.-%,
b. at least one gelling agent selected from the group consisting of homopolymers of acrylic acids, copolymers of acrylic acids, cellulose, cellulose derivatives and polysaccharides, in a total content of more than 5 to 50 wt.-%,
c. at least one emulsifier,
d. at least one fat or wax component which is solid at 25 °C,
e. at least one moisturizer, and
f. 30 wt.-% or less of water,
wherein all contents relate to the total skin-care drying composition.

2. The skin-care drying composition according to claim 1, comprising:
a. 10 to 60 wt.-% of said at least one oil component,
b. 10 to 35 wt.-% of said at least one gelling agent,
c. 5 to 30 wt.-% of said at least one emulsifier,
d. 5 to 25 wt.-% of said at least one fat or wax component
e. 5 to 35 wt.-% of said at least one moisturizer, and
f. less than 1 wt.-% of water.

3. The skin-care drying composition according to claim 2, comprising:
a. 20 to 40 wt.-% of said at least one oil component,
b. 15 to 25 wt.-% of said at least one gelling agent,
c. 5 to 15 wt.-% of said at least one emulsifier,
d. 10 to 20 wt.-% of said at least one fat or wax component, and
e. 10 to 25 wt.-% of said at least one moisturizer.

4. The skin-care drying composition according to any one of the preceding claims, further comprising:
g. at least one cosmetically acceptable alcohol.

5. The skin-care drying composition according to claim 4, comprising:
a. 7 to 20 wt.-% of said at least one oil component,
b. 6 to 15 wt.-% of said at least one gelling agent,
c. 2 to 10 wt.-% of said at least one emulsifier,
d. 3 to 10 wt.-% of said at least one fat or wax component,
e. 2 to 10 wt.-% of said at least one moisturizer, and
g. 5 to 65 wt.-% of said at least one alcohol, wherein said alcohol is a monohydric alcohol.

6. The skin-care drying composition according to any one of the preceding claims, wherein said oil component a. is one or more selected from the group consisting of plant oils and fatty acid ester compounds.

7. The skin-care drying composition according to any one of the preceding claims, wherein said gelling agent b. is one or more selected from the group consisting of polysaccharides and homopolymers of acrylic acids.

8. The skin-care drying composition according to any one of the preceding claims, wherein said emulsifier c. is one or more selected from the group consisting of mono- or difatty acid esters of glycerin, polyglyceryl fatty acid esters, lecithins and modified lecithins.

9. The skin-care drying composition according to any one of the preceding claims, wherein said fat or wax component d. is at least one selected from the group consisting of fatty alcohols and triglycerides of fatty acids.

10. The skin-care drying composition according to any one of the preceding claims, additionally comprising one or more selected from the group consisting of an antibacterial agent, an antiviral agent and an antifungal agent.

11. The skin-care drying composition according to any one of the preceding claims, wherein the composition additionally comprises one or more selected from the group consisting of coenzymes, vitamins, provitamins, pH regulating substances, fragrances, preservatives and UV protectors.

12. The skin-care drying composition according to any one of the preceding claims, wherein the composition comprises:
a. one or more oil component selected from the group consisting of vegetable oils, such as jojoba oil, grape seed oil, apricot kernel oil, sesame oil, coconut oil, babassu oil, olive oil, avocado oil, almond oil, argan oil, aloe vera oil, candula oil, evening primrose oil, fatty acid esters, such as isoamyl laurate and isopropy myristate,
b. one or more gelling agent selected from the group consisting of xanthan gum, guar gum, agar-agar, locus bean gum, and carbomers,
c. one or more emulsifier selected from the group consisting of lecithin, modified lecithins, glyceryl monostearate and glyceryl distearate,
d. one or more fat or wax component selected from the group consisting of lanolin, cetyl alcohol, shea butter, cupuaca butter, cacao butter, lama cream and mango butter,
e. one or more moisturizers selected from the group consisting of polyols such as glycerol, 1,2-propanediol, sorbitol, hyaluronic acid and ceramides, and urea, - optionally one or more alcohol selected from the group consisting of ethanol, propanol, isopropanol and 1-tetradecanol.

13. Use the skin-care and drying composition according to any one of claims 1 to 12 for drying the skin or drying and disinfecting the skin.

14. A non-medical method of washing and drying hands, comprising:
- washing the hands,
- applying the composition according any one of claims 1 to 12 to the wet hands, and
- drying the composition by exposure to air.

15. The method according to claim 14, which does not comprise a step of drying the hands with a towel and/or a step of drying the hands using a device for generating a forced air stream.
